# EUROPEAN PATENT SPECIFICATION

(11) **EP 0 882 797 B1**
(45) Date of publication and mention of the grant of the patent: **16.07.2003**
(21) Application number: 98201288.2
(22) Date of filing: 21.04.1998
(51) Int. Cl.: C12P 7/64, C11C 3/08

(54) **Preparation of symmetrical triglycerides aba**
Herstellung von symmetrischen Triglyceriden aba
Préparation de triglycérides symétriques aba

(30) Priority: 04.06.1997 EP 97201677
(43) Date of publication of application: 09.12.1998
(73) Proprietor: LODERS CROKLAAN B.V., 1521 AZ Wormerveer (NL)
(72) Inventor: Bornscheuer, Uwe T., 70569 Stuttgart (DE); Soumanou, Mohamed M., 70569 Stuttgart (DE); Schmid, Rolf D., 70569 Stuttgart (DE); Schmid, Ulrike, 1520 AA Wormerveer (NL)
(74) Representative: Stevens, Ian Edward

(56) References cited:
- EP-A- 0 185 524
- CHEMICAL ABSTRACTS, vol. 111, no. 1, 3 July 1989 (1989-07-03) Columbus, Ohio, US; abstract no. 6226, KOKUSHO, SUMITAKA ET AL: "Symmetric triglycerides and their manufacture with high molecular weight lipase" XP002143781 & JP 63 240790 A (SHOKUHIN SANGYO BIOREACTOR SYSTEM GIJUTSU KENKYU KUMIAI, JAPAN) 6 October 1988 (1988-10-06)
- DATABASE WPI Section Ch, Week 198846 Derwent Publications Ltd., London, GB; Class D16, AN 1988-326763 XP002143783 & JP 63 240790 A (SHOKUHIN SANGYO BIOREACTOR), 6 October 1988 (1988-10-06)
- BLOOMER, S.: "Lipase-catalysd lipid modifications in non-aqueous media." DISSERTATION ABSTRACTS INTERNATIONAL, vol. 53, no. 4, 1992, page 730 XP002143779 Sweden
- CHEMICAL ABSTRACTS, vol. 108, no. 23, 6 June 1988 (1988-06-06) Columbus, Ohio, US; abstract no. 203583, ABE, MASAYUKI ET AL: "Manufacture of cacao butter substitutes by ester exchange reaction of fat and oils using lipase" XP002143782 & JP 62 228290 A (KANEGAFUCHI CHEMICAL INDUSTRY CO., LTD., JAPAN) 7 October 1987 (1987-10-07)
- DERKSEN, J. T. P. AND CUPERUS F. P.: "Lipase-catalyzed hydrolysis of crambe oil in AOT-isooctane reversed micelles." BIOTECHNOLOGY LETTERS, vol. 14, no. 10, 1992, pages 937-940, XP002143780
- BAYON Y ET AL: "Two step synthesis of 1,3-acetylated, -butyroylated and -caproylated triglycerides from a microorganism oil rich in Docosahexaenoic Acid (DHA)" BIOORGANIC & MEDICINAL CHEMISTRY LETTERS,GB,OXFORD, vol. 6, no. 4, 20 February 1996 (1996-02-20), pages 345-348, XP004135033 ISSN: 0960-894X
- MILLQVIST A. ET AL: 'Lipase-catalysed alcoholysis of triglycerides for the preparation of 2-monoglycerides.' ENZYME MICROB. TECHNOL. vol. 16, 1994, pages 1042 - 1047
- GUNSTONE F.D. ET AL: 'THE LIPID HANDBOOK', 1986, CHAPMAN AND HALL, LONDON NEW YORK Pages 77 and 100

## Description

Symmetrical triglycerides of the type ABA, wherein A and B both can be selected from saturated and unsaturated fatty acid residues are known compounds in the art. Typical examples of these triglycerides that are applied at industrial scale are OPO as human milk fat replacer, SOS as cocoa butter equivalent, C12C18C12- or C18C12C18-triglycerides as antiblooming agent or as structuring component in spreads. All these components are made so far by performing a chemical or enzymic esterification on an oil or a fraction thereof. However all these reactions resulted in products wherein the amount of B originally present in the 2-position of the triglyceride was decreased as a result of the esterification reaction.

Although JP 62/228290 (published Oct '87) discloses a method for the preparation of cocoa butter substituents, wherein in a first step a fat high in 2-oleic triglycerides is subjected to an enzymatic hydrolysis resulting in a product high in 2-monoglycerides, whereupon this product is resynthesised using a 1,3 specific lipase to a product rich in SOS type triglycerides, it can also be concluded from the results mentioned in this patent that the hydrolysis never was performed in such a way that the product obtained was a pure or nearly pure 2-oleic monoglyceride. From the example it can be concluded that starting from a palm fraction and using stearic acid for the resynthesis the product obtained contained appreciable amounts of triglycerides with palmitic in the 1,3 positions. This can only be explained by the fact that the hydrolysis never led to a product high in 2-oleic monoglyceride. This product must have contained appreciable amounts of partial glycerides also containing palmitic acid in it (such as the diglycerides PO or the P-monoglyceride),which upon further reaction might have isomerised. A confirmation herefore can also be found in the fact that the total of triglycerides POP + POS + SOS in the reaction product only adds up to about 80% sothat still
20% of other not identified impurities must have been present.
Above might be due to the fact that the hydrolysis is performed in the presence of excess of water.

According to JP 63240790 symmetrical triglycerides can be made by an enzymic conversion of 2-mono glycerides with a source for fatty acid moieties to be incorporated in the 1 and 3 positions of the glyceride by using high molecular weight enzymes.It is specifically indicated that the use of other enzymes do not lead to acceptable results e.g. because of the fact that the other enzymes are inactive or deactivate rapidly.We now found a process wherein these other enzymes (lipases) could be applied succesfully. Bloomer ,cf Dissertation Abstracts International 53,4,1992,p.730 discloses the preparation of symmetrical triglycerides along an enzymic route by converting a triglyceride with an acyldonor using a 1,3 specific enzyme.Although it is indicated that 2-mono glycerides can be made , it does not reveal the conversion thereof into symmetrical triglycerides.
Derksen in Biotechn Letters 14,10,1992,p.937 reveals that crambe oil is a good starting material for the production of hydrolysis products.EP 185524 sets out the impact of the A_{w} on the production of diglycerides in enzymic conversions.

We performed a study in order to find a process wherein an optimal use could be made of the amount of fatty acid B that was present in the starting material and in which the level of isomerisation was limited to the minimum , while using commercially available 1,3 specific enzymes. The process we found is based on the use of monoglycerides with a high level of 2-B-monoglyceride in esterification processes wherein we could avoid that the group B migrated over the glycerol backbone during the conversion of the 2-B-monoglyceride into triglycerides of the ABA type.
This was highly unexpected because it is well documented in the prior art that partial glycerides including monoglycerides are easily isomerised during their conversion, in particular if the conversion is performed in the presence of water or traces of acid or base.

We further studied whether the type of support for the enzyme had an impact on the end product composition and whether the presence of a solvent would influence the product composition. We found that in all instances such an impact existed.

Therefore our invention concerns in the first instance a process for the preparation of triglycerides of the ABA type, wherein A = saturated or unsaturated or polyunsaturated fatty acid residue with 2-24 C atoms and B = saturated or unsaturated or polyunsaturated fatty acid residue, while A is not simultaneously identical with B in the same molecule by:
subjecting a compound with a content of more than 80 % of, most preferably more than 90 % of 2-monoglyceride with a group B substituted at the 2 position to an enzymatic conversion in the absence of a solvent with a reactant providing fatty acid moieties A using a 1,3-specific enzyme selected from the group consisting of Rhizopus delemar, Rhizopus javanicus and Rhizomucor miehei under conditions that control the water activity Aw to a value < 1.0, in particular 0.1-0.5.

Essential for the positive outcome are thus the availability of a monoglyceride with a high content of 2-B-monoglyceride and the conversion hereof with a 1,3 specific enzyme under well controlled conditions of water content. In particular the water that is formed during the conversion must be removed during the enzymatic conversion, so that the Aw value during the conversion is maintained at a value < 1.0, in particular between 0.1 and 0.5. Herefore the reaction can be performed in the presence of a water absorbent such as a molecular sieve or the water can be removed by using reduced pressure and elevated temperature during the conversion.

The best results were obtained if the enzymes were applied as immobilised enzyme on a support material, preferably selected from celite^{R}, silanised celite^{R}, alumina, silica, ion exchange resins, hydrophobic supports in particular polymerisation products from ethylene, or propylene, or styrene etc and most particular those known as EP 100^{R} (an AKZO product).
However we found that the nature of the support used for the 1,3 specific enzyme used for the synthesis of the symmetrical triglyceride ABA from the 2-B monoglyceride had a high impact on both yield of the end product and on the purity of the endproduct ABA. Hydrophobic supports like EP 100^{R} (ie a polypropylene support from AKZO ) were found to give the highest yields of the purest ABA in the shortest times. Therefore we prefer to apply an 1,3 specific enzyme supported on a hydrophobic support for the conversion of the 2-B monoglyceride into the symmetrical triglyceride ABA. These hydrophobic supports are well known from eg our EP 322 213
We found that the highest yields of the purest products were obtained if the conversion of 2-B monoglyceride was performed in the absence of a solvent.

The reactant providing the fatty acid moieties A that will react with the 2-B-monoglycerides can be selected from the group consisting of free fatty acids, alkyl esters of free fatty acids, in particular those with 1-6 C atoms in the alcohol part of the ester chain and in particular having an activated ester group and triglyceride. For the purposes of the invention it is a prerequisite that the triglyceride compounds have high contents of fatty acid residues A in the 1 and 3 positions. Typical examples of these components are palm top fraction (ie rich in PPP), olive oil and other high oleic vegetable oils (ie rich in 03), cocoa butter fats (ie rich in saturated C16/C18 fatty acid residues). However with the use of free fatty acids or their alkyl esters it is often easier to achieve the most optimal results.

Although the process can be performed in the presence of a solvent we found that the best results are obtained if a solvent is absent during the conversion. A very suitable solvent however, is the short alkyl ester of the free fatty acid that can also act as the source for the fatty acids A. Conveniently the conversion is performed using mole ratios monoglyceride : reactant providing fatty acid moiety A of 1:2 to 1:4.

The process is very well applicable for the production of the following triglycerides from the materials indicated, ie:
i) a 2-palmitate monoglyceride with an oleic acid source to prepare OPO rich fats.
ii) a 2-oleic monoglyceride with a source for C16 and/or C18 fatty acids to prepare SOS fats. (S= palmitic or stearic)
iii) a 2-saturated monoglyceride having either 10-14 or 16-24 C atoms in the fatty acid residue with a fatty acid source having either 16-24 or 10-14 C atoms to prepare symmetrical saturated triglycerides of the ABA type.

The compound with the high content of 2-B-monoglyceride can be made by a prior art process. This process comprises an alcoholysis of a triglyceride with a high percentage of B bonded in the 2-position, using a 1,3 specific enzyme in the presence of an organic solvent while maintaining the wateractivity during the conversion at 0.05-1.0, preferably 0.4-0.9. This process is known from Millqvist c.s. Enzyme Microb Technol 16 (1994) 1042-1047 and Biocatalysis 14 (1996) 89-111.

Again it was found that on the hydrolysis the nature of the support material for the enzyme had a high impact on the product composition. It was found that by using an enzyme on the same hydrophobic supports as mentioned above the highest yields of the purest 2-B monoglycerides could be obtained.

The 2-B-monoglyceride is isolated from the crude reaction mixture by crystallisation at a suitable temperature, ie low enough to achieve the desired results, such as T < -10 oC, for 2-unsaturated monoglycerides.
The organic solvent that is applied is selected from the group consisting of hydrocarbons, preferably having 4-12 C atoms, ethers, in particular having at least one branched alkyl residue, ketones, in particular acetone and alcohols.

The alcoholysis is in particular performed in a reaction system with a mole ratio triglyceride : alcohol of 1:5 to 1: 20.

### EXPERIMENTAL PART

### MATERIALS AND METHODS

Lipase. Lipases (triacylglycerol lipases, EC 3.1.1.3) were from *Rhizopus delemar* (RDL), *Rhizopus javanicus* (RJL) (both from Amano Pharmaceutical Co. Ltd, Nagoya, Japan) and from *Rhizomucor miehei* (RML) (Biocatalysts Ltd, Pontybridd, England). One commercial lipase from RML (Lipozyme^{R} IM), immobilized on anion exchange resin, was from Novo (Bagsvaerd, Denmark). All chemicals and solvents used were reagent grade and purchased from common commercial suppliers, except peanut oil (Vaselin Fabrik, Bonn, Germany), EP 100^{R} (porous polypropylene particles, 200-400 µm) (Akzo, Obernburg, Germany), and Celite 545^{R} (Fluka, Buchs, Switzerland).
Hydrolytic activity of lipase. The hydrolytic activity of lipases was measured with 5% (w/v) olive oil emulsion (pH 8.0) containing 2% (w/v) gum arabic at 37°C. 20 ml of the emulsified solution, 470 µl of CaCl₂ solution (22% w/v) and a known amount of immobilized lipase were mixed, and liberated fatty acids were titrated automatically with 0.01 N NaOH in order to maintain the pH constant at pH 8.0. One unit (U) of lipase activity was defined as the amount of enzyme, which liberates 1 µmol fatty acid per min under assay conditions.
Immobilization of lipase. Celite^{R} 545 and EP 100^{R} were used as supports for adsorptive immobilization of commercial lipases. Before immobilization, celite^{R} was washed several times with purified water and ethanol (95% v/v) to remove fines, and dried at 80°C. Then the pre-washed celite^{R} was mixed with 5% HNO₃ and stirred at 80°C for 4 hours. The acid-washed celite^{R} was carefully washed with purified water until the pH of the water was neutral and was then dried overnight at 80°C. One gram lipase powder was dissolved in 20 ml phosphate buffer (pH 7.0, 20 mM). The solution was added to 1.5 g acid-washed celite^{R} or EP 100^{R} stirred at 5°C for 8 hours followed by addition of 5 ml chilled acetone (-15°C). The immobilized lipase was collected by filtration, washed three times with phosphate buffer (pH 7.0, 20 mM), dried at room temperature under vacuum for 48 hours and stored at 5°C until use.

### EXAMPLE 1

### Alcoholysis of triacylglycerides

Triolein (99%) or trilinolein (99%) (both 0.28 M) dissolved in methyl *tert*-butyl ether (MTBE) (2 ml) was equilibrated to water activity of 0.75 over saturated NaCl in a closed vessel. Dry ethanol was added to a final concentration of 2.8 M. The reaction mixture was incubated at 40°C in a stirred oil bath for 15 min, then the reaction was started by addition of 60 mg of immobilized lipase from RML, RDL or RJL, pre-equilibrated at water activity 0.11 over saturated LiCl. The reaction was stopped after 30 hours by removal of immobilized lipase. After evaporation of excess solvent *in vacuo*, the oily residue was dissolved in 20 ml *n*-hexane:MTBE (70:30 v/v) and stored at -25°C overnight. After this period, white crystals formed, which were collected by filtration at -25°C. The supernatant containing ethyl esters, diacylglycerides (DG), fatty acids and a small amount of TG was discarded. The 2-MG was recrystallized several times until the TLC plate showed only one band of 2-MG. Yields given in Tables 1 and 2 are percentages of the theoretical yield of 33.3 %. The purity of 2-MG was confirmed by NMR spectroscopy.

**Table 1:**

| **1)** Synthesis of 2-monoolein (2-MO) by alcoholysis of triolein with ethanol in MTBE with lipases immobilized on Celite | | | |
|---|---|---|---|
| Lipase from | Initial rate (µmol/h/mg) | Initial rate (µmol/h/U) | Yield (%) |
| *Rhizomucor miehei* | 0.56 | 2.23 | 40.5 |
| | | | |
| *Rhizopus delemar* | 0.65 | 0.30 | 71.8 |
| | | | |
| *Rhizopus javanicus* | 0.82 | 0.25 | 56.9 |

**Table 2:**

| Influence of triacylglyceride on the synthesis of 2-MG by alcoholysis with immobilized lipase from *Rhizopus delemar* in MTBE and ethanol. | | |
|---|---|---|
| Triglyceride | Initial rate (µmol/h/U) | Yield (%) |
| Triolein | 0.30 | 71.8 |
| | | |
| Trilinolein | 0.41 | 60.6 |

### EXAMPLE 2

### Esterification reaction

The purified 2-mono olein obtained by alcoholysis in example 1 (60 mM) and caprylic acid (180 mM) were dissolved in 2 ml of *n*-hexane equilibrated to a water activity of 0.11. The reaction was started by addition of 12% (based on the weight of 2-MO) of different immobilized lipases **(water activity 0.11)** and 0.5 gram molecular sieve (4 Å). An aliquot of the reaction medium was applied to a Kieselgel 60^{R} plate, which was developed in a mixture of n-hexane:diethyl ether:acetic acid (70:30:1.5). The components of the reaction mixture were visualized by spraying with 50% (v/v) sulfuric acid (dissolved in methanol), and heated at 150°C.

### Purification of produced TG

The reaction mixture was purified by column chromatography. Silica gel^{R} (10g) and aluminium oxide (10g) were mixed in 50 ml n-hexane to make a slurry, which was poured into the column (300 mm x 30 mm). The reaction mixture (1g) containing DG, fatty acids and produced TG was applied to the column. The column was eluted with a mixture of n-hexane:diethyl ether (95:5 v/v). The recovered fractions were analysed by TLC.

### HPLC separation of triacylglycerols

The composition of the triacylglycerols formed during the enzymatic esterification and interesterification was characterised by HPLC using a nucleosil^{R} C₁₈ column, (5µm, 250 x 4 mm, Sykam, Gilching, Germany) and an evaporative light scattering detector (S.E.D.E.R.E, Vitry/Seine, France) at a column temperature of 38°C and a flow rate of 1.0 ml/min. Elution was performed using a linear gradient elution system of 80% acetonitrile to 90% dichloromethane over 40 min. The extent of reaction was calculated from the molar or weight percentage of produced triacylglycerides present in the mixture. The results are given in Table 3.

**Table 3:**

| 2)Synthesis of structured triacylglyceride by esterification of 2-mono olein with caprylic acid (1:3 molar ratio) in *n*-hexane with different immobilized lipases. | | | | | |
|---|---|---|---|---|---|
| Lipase | Support | Initial rate [µmol/h/U] | | Concentration of TG [wt%] | |
| | | MLM | MLL | MLM | MLL |
| RML | Resin | 70.6 | 3.7 | 89.1 | 8.8 |
| | | | | | |
| RDL | EP 100 | 3.1 | <0.1 | 91.2 | 8.0 |
| M = caprylic acid | | | | | |
| L = oleic acid | | | | | |

### EXAMPLE 3

### Alcoholysis of triglycerides to yield 2-palmitic monoglycerides

Tripalmitin (=palm top fraction) was subjected to alcoholysis using Rhizopus Delamar (from Amano, Nagoya, Japan) in acetone. The water activity was maintained at about 0.43. The reaction was performed as described before in example 1 except that acetone was applied as solvent and EP 100^{R} was applied as support for the enzyme. The product (2-palmitic monoglyceride) was obtained in a yield of 90 % and had a purity of >95 %.

### EXAMPLE 4

### Esterification of 2-palmitic monoglyceride

The 2-palmitic monoglyceride of example 3 was converted with oleic acid in a mole ratio of 1:3 using Rhizopus delamar on either Celite^{R} or on EP 100^{R} as support material. Water formed during the conversion was removed in vacuo. The reaction was performed in the absence of a solvent (for the EP^{R} 100) or in the presence of n-hexane (in case Celite^{R} was applied as support).
The following results were obtained:

| support | solvent | time | yield | P in 2 position of product OPO |
|---|---|---|---|---|
| Celite^{R} | hexane | 24 hr | 72% | 94 % |
| | | | | |
| EP 100^{R} | no | 16 hr | 78% | 95.9 % |

## Claims

1. Process for the preparation of triglycerides of the ABA type, wherein A = saturated or unsaturated or polyunsaturated fatty acid residue with 2-24 C atoms and B = saturated or unsaturated or polyunsaturated fatty acid residue, while A is not simultaneously identical with B in the same molecule by:
subjecting a compound with a high content of more than 80 % of, most preferably more than 90 % of 2-monoglyceride with a group B substituted at the 2 position to an enzymatic conversion in the absence of a solvent with a reactant providing fatty acid moieties A using a 1,3-specific enzyme selected from the group consisting of Rhizopus species, preferably Rhizopus delemar, Rhizopus javanicus or Rhizomucor meihei under conditions that control the water activity Aw to a value < 1.0, in particular 0.1-0.5.

2. Process according to claim 1 wherein the enzyme is supported on a hydrophobic support material

3. Process according to claims 1-2, wherein the Aw is controlled by removal of the water formed during the enzymatic conversion, in particular by removal at elevated temperature at reduced pressure and/or adsorption of water by an absorbent.

4. Process according to claims 1-3, wherein the reactant providing the fatty acid moieties A is selected from the group consisting of free fatty acids, alkyl esters of free fatty acids with 1-6 C atoms in the alcoholic residue and triglycerides.

5. Process according to claims 1-4, wherein the process is performed in the presence of alkyl esters of the fatty acids that can act as reactant.

6. Process according to claims 1-5, wherein a mole ratio monoglyceride : reactant providing fatty acid moiety A of 1:2-1:4 is applied.

7. Process according to claims 1-6, wherein one of the following conversions is performed:
i)a 2-palmitate monoglyceride with an oleic acid source to prepare OPO rich fats ( O = oleic acid;P = palmitic acid).
ii)a 2-oleic monoglyceride with a source for C16 and/or C18 fatty acids to prepare SOS fats ( S = palmitic or stearic acid ).
iii)a 2-saturated monoglyceride having either 10-14 or 16-24 C atoms in the fatty acid residue with a fatty acid source having either 16-24 or 10-14 C atoms to prepare symmetrical saturated triglycerides of the ABA type.

8. Process according to claims 1-7, wherein the compound with the high 2-B-monoglyceride content is obtained by enzymatic alcoholysis of a triglyceride with a high percentage of B bonded in the 2-position, using a 1,3 specific enzyme in the presence of an organic solvent while maintaining the water activity during the conversion at 0.05-1.0.

9. Process according to claim 8 wherein the enzyme applied for the hydrolysis is supported on a hydrophobic support material.

10. Process according to claim 8, wherein the 2-B monoglyceride product formed is isolated from the crude reaction mixture by crystallisation at < -10 oC.

11. Process according to claim 8, wherein the organic solvent is selected from the group consisting of hydrocarbons, ethers, ketones, alcohols.

12. Process according to claim 8, wherein the alcoholysis is performed on a reaction system with a mole ratio triglyceride: alcohol of 1:5-1:20.

## Patentansprüche

1. Verfahren zur Herstellung von Triglyceriden des Typs ABA, worin A = gesättigter oder ungesättigter oder mehrfach ungesättigter Fettsäurerest mit 2 bis 24 C-Atomen und B = gesättigter oder ungesättigter oder mehrfach ungesättigter Fettsäurerest, wobei A im selben Molekül nicht mit B identisch ist, indem eine Verbindung mit einem Gehalt von mehr als 80 %, bevorzugter mehr als 90 % an 2-Monoglycerid mit einer Gruppe B, substituiert in der 2-Position, einer enzymatischen Umwandlung in Abwesenheit eines Lösungsmittels mit einem Reaktanten, der Fettsäuregruppierungen A bereitstellt, unter Verwendung eines 1,3-spezifischen Enzyms, ausgewählt aus der Gruppe bestehend aus Rhizopus-Species, vorzugsweise Rhizopus delemar, Rhizopus javanicus oder Rhizomucor meihei, unter solchen Bedingungen, dass die Wasseraktivität Aw auf einen Wert < 1,0, insbesondere 0,1 bis 0,5, reguliert wird, unterzogen wird.

2. Verfahren gemäß Anspruch 1, wobei das Enzym auf einem hydrophoben Trägermaterial getragen wird.

3. Verfahren gemäß Anspruch 1 oder 2, wobei die Aw durch Entfernung des Wassers, das während der enzymatischen Umwandlung gebildet wird, insbesondere durch Entfernung bei erhöhter Temperatur bei reduziertem Druck und/oder Adsorption von Wasser durch ein Absorptionsmittel, reguliert wird.

4. Verfahren gemäß den Ansprüchen 1 bis 3, wobei der Reaktänt, der die Fettsäuregruppierungen A liefert, aus der Gruppe bestehend aus freien Fettsäuren, Alkylestern von freien Fettsäuren mit 1 bis 6 C-Atomen im Alkoholrest und Triglyceriden ausgewählt wird.

5. Verfahren gemäß den Ansprüchen 1 bis 4, wobei das Verfahren in Gegenwart von Alkylestern der Fettsäuren, die als Reaktanten wirken können, durchgeführt wird.

6. Verfahren gemäß den Ansprüchen 1 bis 5, wobei ein Molverhältnis Monoglycerid : Reaktant, der die Fettsäuregruppierung A liefert, von 1:2-1:4 angewendet wird.

7. Verfahren gemäß den Ansprüchen 1 bis 6, wobei eine der folgenden Umwandlungen durchgeführt wird:
i) ein 2-Palmitat-monoglycerid mit einer Ölsäurequelle unter Herstellung OPO-reicher Fette (O = Ölsäure; P = Palmitinsäure);
ii) ein 2-Ölsäuremonoglycerid mit einer Quelle für C16-und/oder C18-Fettsäuren unter Herstellung von SOS-Fetten (S = Palmitin- oder Stearinsäure);
iii) ein 2-gesättigtes Monoglycerid mit entweder 10-14 oder 16-24 C-Atomen im Fettsäurerest mit einer Fettsäurequelle, die 16-24 oder 10-14 C-Atome hat, unter Herstellung symmetrischer gesättigter Triglyceride des Typs ABA.

8. Verfahren gemäß den Ansprüchen 1 bis 7, wobei die Verbindung mit dem hohen 2-B-Monoglyceridgehalt durch enzymatische Alkoholyse eines Triglycerids mit einem hohen prozentualen Anteil von B, in der 2-Position gebunden, unter Verwendung eines 1,3-spezifischen Enzyms in Gegenwart eines organischen Lösungsmittels, während die Wasseraktivität der Umwandlung bei 0,05 bis 1,0 gehalten wird, erhalten wird.

9. Verfahren nach Anspruch 8, wobei das für die Hydrolyse angewendete Enzym auf einem hydrophoben Trägermaterial getragen wird.

10. Verfahren nach Anspruch 8, wobei das gebildete 2-B-Monoglycerid-Produkt aus dem rohen Reaktionsgemisch durch Kristallisation bei < -10 °C isoliert wird.

11. Verfahren nach Anspruch 8, wobei das organische Lösungsmittel aus der Gruppe bestehend aus Kohlenwasserstoffen, Ethern, Ketonen, Alkoholen ausgewählt wird.

12. Verfahren nach Anspruch 8, wobei die Alkoholyse an einem Reaktionssystem mit einem Molverhältnis Triglycerid : Alkohol von 1:5-1:20 durchgeführt wird.

## Revendications

1. Procédé pour la préparation de triglycérides de type ABA, dans lequel A = résidu d'acide gras saturé ou insaturé ou poly-insaturé avec 2 à 24 atomes de C et B = résidu d'acide gras saturé ou insaturé ou poly-saturé, A n'étant pas simultanément identique à B dans la même molécule en :
soumettant un composé avec une teneur supérieure à 80%, de préférence supérieure à 90% en monoglycéride-2 présentant un groupe B substitué en position 2, à une conversion enzymatique en l'absence d'un solvant en présence d'un réactif fournissant des fragments d'acide gras A en utilisant une enzyme 1,3-spécifique sélectionnée dans le groupe constitué d'espèces Rhizopus, de préférence Rhizopus delemar, Rhizopue javanicus ou Rhizomucor meihei, dans des conditions qui contrôlent l'activité de l'eau Aw selon une valeur < 1,0, plus particulièrement située entre 0,1 et 0,5.

2. Procédé selon la revendication 1, dans lequel l'enzyme est portée sur un matériau de support hydrophobe.

3. Procédé selon les revendications 1 à 2, dans lequel le Aw est contrôlé par le retrait de l'eau formée pendant la conversion enzymatique, plus particulièrement par retrait à une température élevée à pression réduite et/ou par adsorption de l'eau par un absorbant.

4. Procédé selon les revendications 1 à 3, dans lequel le réactif fournissant les fragments d'acide gras A est sélectionné dans le groupe constitué d'acides gras libres, esters alkyliques d'acides gras libres avec 1 à 6 atomes de C dans le résidu alcoolique et triglycérides.

5. Procédé selon les revendications 1 à 4, dans lequel le procédé est réalisé en présence d'esters alkyliques des acides gras qui peuvent agir comme réactifs.

6. Procédé selon les revendications 1 à 5, dans lequel un rapport molaire monoglycéride : réactif fournissant un fragment d'acide gras A, de 1:2 à 1:4 est appliqué.

7. Procédé selon les revendications 1 à 6, dans lequel l'une des conversions suivantes est effectuée :
i) monoglycéride de palmitate-2 avec une source d'acide oléique afin de préparer des graisses riches en OPO (O = acide oléique ; P = acide palmitique).
ii) monoglycéride oléique avec une source d'acides gras C16 et/ou C18 afin de préparer des graisses de SOS (S = acide palmitique ou stéarique).
iii) monoglycéride2-saturé possédant 10 à 14 ou 16 à 24 atomes de C dans le résidu d'acide gras avec une source d'acide gras possédant 16 à 24 ou 10 à 14 atomes de C, afin de préparer des triglycérides saturés symétriques de type ABA.

8. Procédé selon les revendications 1 à 7, dans lequel le composé avec la teneur élevée en monoglycéride-2-B est obtenu par alcoolyse enzymatique d'un triglycéride avec un pourcentage élevé de B lié en position 2, en utilisant une enzyme 1,3-spécifique en présence d'un solvant organique, tout en maintenant l'activité de l'eau pendant la conversion à 0,05 - 1,0.

9. Procédé selon la revendication 8, dans lequel l'enzyme appliquée pour l'hydrolyse est portée sur un matériau de support hydrophobe.

10. Procédé selon la revendication 8, dans lequel le produit de monoglycéride-2-B formé est isolé du mélange réactionnel brut par cristallisation à < -10°C.

11. Procédé selon la revendication 8, dans lequel le solvant organique est sélectionné dans le groupe constitué d'hydrocarbures, d'éthers, de cétones, d'alcools.

12. Procédé selon la revendication 8, dans lequel l'alcoolyse est effectuée sur un système de réaction avec un rapport molaire triglycéride : alcool, de 1:5 à 1:20.
